(19) Europäisches Patentamt
European Patent Office
Office européen des brevets

(11) Publication number: **0 268 369 B1**

(12) **EUROPEAN PATENT SPECIFICATION**

(45) Date of publication of patent specification: **14.08.91**

(51) Int. Cl.⁵: **C07D 403/14**, A61K 31/495

(21) Application number: **87309018.7**

(22) Date of filing: **13.10.87**

# (54) Bridged bicyclic imides as anxiolytics and antidepressants.

(30) Priority: **21.10.86 PCT/US86/02224**

(43) Date of publication of application:
**25.05.88 Bulletin 88/21**

(45) Publication of the grant of the patent:
**14.08.91 Bulletin 91/33**

(84) Designated Contracting States:
**AT BE CH DE ES FR GB GR IT LI LU NL SE**

(56) References cited:
**EP-A- 0 200 968**
**DE-A- 2 920 739**
**DE-A- 3 529 872**
**US-A- 4 562 255**

**CHEMICAL ABSTRACTS, vol. 103, no. 25, December 23, 1985, Columbus, Ohio, US; SUMITOMO CHEMICAL Co. Ltd.: "Piperazinylalkylsuccinimides", page 898, column 1, abstract no. 215 313k**

**CHEMICAL ABSTRACTS, vol. 101, no. 21, November 19, 1984, Columbus, Ohio, US; SUMITOMO CHEMICAL Co. Ltd.: "Cyclic imide derivatives", page 776, column 1, abstract-no. 191 971d**

(73) Proprietor: **PFIZER INC.**
**235 East 42nd Street**
**New York, N.Y. 10017(US)**

(72) Inventor: **Bright, Gene Michael**
**329, Tyler Avenue**
**Groton, CT 06340(US)**

(74) Representative: **Bradbrook, Geoffrey William et al**
**PFIZER LIMITED Ramsgate Road**
**Sandwich Kent, CT13 9NJ(GB)**

**EP 0 268 369 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Anxiety and depression are common afflictions which adversely affect a significant portion of the human population. Both anxiety and depression can appear as either acute or chronic disease states, and in certain subjects these disease states can co-exist.

It has been known for many years that the symptoms of anxiety and depression in human subjects can often be alleviated by the administration of certain chemical substances. In this regard, compounds which are used to treat anxiety are called antianxiety agents, or anxiolytics; while compounds which are used to treat depression are normally termed antidepressants.

In modern medical practice, a widely-used class of anxiolytics is the benzodiazepines, such as diazepam, and common antidepressants are the so-called "tricylics," such as imipramine. However, benzodiazepines also have sedative properties in addition to their antianxiety properties. Moreover, tricyclic antidepressants often exhibit undesirable cardiovascular and anticholinergic side-effects.

Accordingly there is a need for new pharmacologic agents for the treatment of anxiety and depression. In particular, there is a need for anxiolytic agents which do not possess sedative effects; i.e., there is a need for anxiolytics which exhibit selectivity of action.

Certain glutarimide and succinimide compounds, substituted on nitrogen by a (4-aryl-1 piperazinyl)alkyl or (4-heteroaryl-1-piperazinyl)alkyl group, and having tranquillizing, antianxiety and/or anti-emetic properties, are known from United States patents Nos. 3,717,634, 3,907,801, 4,182,763, 4,423,049, 4,507,303 and 4,562,255 and 4,543,355. Korgaonka et. al., J. Indian Chem. Soc., 60, 874 (1983), disclosed a number of N-(3-[4-aryl-1-piperazinyl]propyl) camphorimides, which are alleged to have sedative properties in mice.

This invention relates to new chemical compounds, which possess antianxiety and antidepressant properties. More particularly, the compounds of this invention are bridged bicyclic imide compounds of the formula

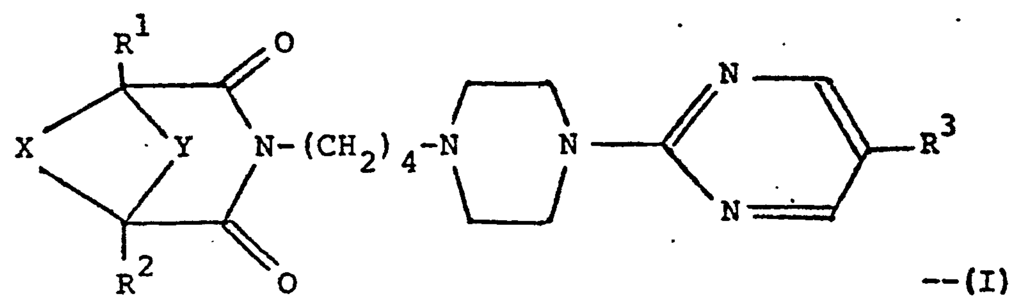

--(I)

and the pharmaceutically-acceptable acid-addition salts thereof, wherein

$R^1$ and $R^2$ are each selected from the group consisting of H and $CH_3$;

$R^3$ is selected from the group consisting of H and F; and either

(a) X is selected from the group consisting of $CH_2$, $CH_2CH_2$ and $CH_2CH_2CH_2$; and Y is selected from the group consisting of $CH_2$, $CH(CH_3)$, $C(CH_3)_2$, $C(CH_2)_4$ (i.e. cyclopentylidene), and $CH_2CH_2$.

or (b) X is selected from the group consisting of $CH=CH$, $CH_2CH(CH_3)$ and $CH_2C(CH_3)_2$; and Y is $CH_2$.

Accordingly, this invention provides: (i) the novel compounds of the formula I and the pharmaceutically acceptable acid-addition salts thereof; and (ii) pharmaceutical composition which comprise a pharmaceutically-acceptable carrier and a compound of formula I or a pharmaceutically-acceptable acid-addition salt thereof.

A preferred group of compounds of the formula I comprises those compounds wherein $R^3$ is hydrogen, X is $CH_2$, $CH_2CH_2$ OR $CH_2CH_2CH_2$ and Y is $CH_2$, $CH(CH_3)$, $C(CH_3)_2$, $C(CH_2)_4$ or $CH_2CH_2$. Compounds in which X is $CH_2CH_2$ are especially preferred. Particularly preferred compounds within this preferred group are those wherein $R^1$ is methyl, $R^2$ is hydrogen and Y is $C(CH_3)_2$. An especially preferred individual compound of this invention is the dextrorotatory isomer of 3-(4-[4-(2-pyrimidinyl)-1-piperazinyl]butyl)-1,8,8-trimethyl-3-azabicyclo[3.2.1.]octan-2,4-dione.

Other preferred compounds are those in which $R^1$ is $CH_3$, $R^2$ is H, $R^3$ is F, X is $CH_2CH_2$ and Y is $C(CH_3)_2$, and those in which $R^3$ is H, X is $CH=CH$, $CH_2CH(CH_3)$ or $CH_2C(CH_3)_2$ and Y is $CH_2$, particularly when $R^1$ and $R^2$ are H and X is $CH_2CH(CH_3)$.

The compounds of this invention are the compounds of the formula I and salts thereof. In one method according to the invention, the compounds of the formula I are prepared by reaction of a cyclic anhydride of the formula II with the requisite amine of the formula III.

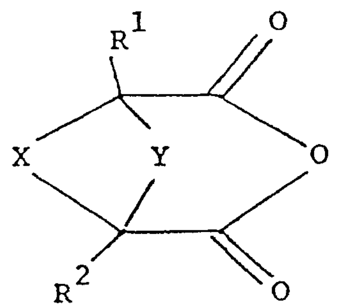

--(II)

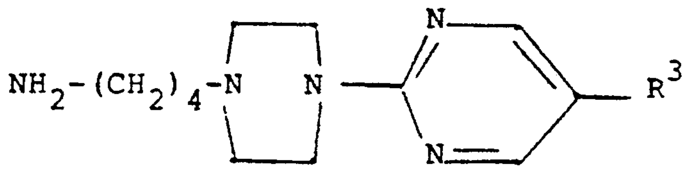

--(III)

wherein $R^1$, $R^2$, $R^3$, X and Y are as defined previously. The reaction between the anhydride II and the amine III is commonly carried out by heating substantially equimolar quantities of the two compounds at a temperature from 90 to 160 °C, until the reaction is substantially complete. The two reactants are usually heated in a reaction-inert solvent; however, in those cases in which one or both of the reactants is molten at the reaction temperature, the two reactants can be heated in the absence of solvent. A reaction-inert solvent is one in which at least one of the reactants is soluble, and which does not adversely interact with either of the starting reactants or the product of the formula I. Typical reaction-inert solvents which can be used include hydrocarbons, such as benzene, toluene, xylene and decalin, and the methyl and ethyl ethers of ethylene glycol, propylene glycol and diethylene glycol. Reaction between an anhydride of the formula II and an amine of the formula III is normally carried out under substantially anhydrous conditions.

Reaction between an anhydride of formula II and an amine of the formula III proceeds more rapidly at high temperatures than lower temperatures, and it proceeds more rapidly in the absence of solvent than when carried out in solution. Thus, in a typical case, reaction of a compound of formula II with a compound of formula III in a reaction-inert solvent at about 120 °C commonly takes several hours e.g., 12 to 30 hours. However, reaction times of as little as about one hour are sufficient if reaction temperatures of about 220-30 °C are used.

If no solvent has been used, the compound of formula I is obtained directly. When a reaction-inert solvent has been used, the compound of formula I is usually recovered by solvent evaporation. A compound of formula I can be purified by standard procedures, such as recrystallization and/or chromatography.

The anhydrides of the formula II are normally prepared by dehydration of the corresponding dicarboxylic acid of the formula IV:

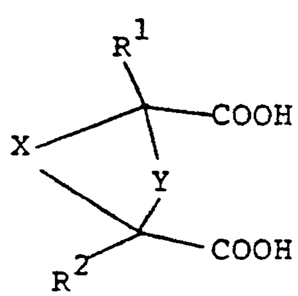

IV

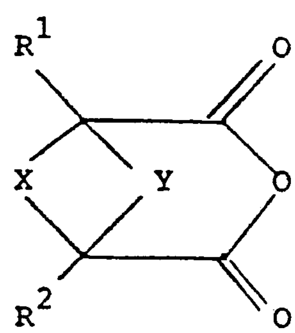

II

wherein $R^1$, $R^2$, X and Y are as defined previously. This dehydration is carried out under standard conditions, well-known for this kind of transformation. For example, in a typical procedure, a dicarboxylic acid of the formula IV is heated under reflux for a few hours, e.g., two to four hours, in a large excess of acetic anhydride. Removal of the volatile materials by evaporation in vacuo then affords the anhydride of formula II.

The dicarboxylic acids of the formula IV are either known compounds, which are prepared by the known procedures, or they are analogs of known compounds, which are prepared by methods analogous to the known procedures. Methods which are available for preparing dicarboxyclic acids of the formula IV include ozonolysis of an olefin of the formula V, nitric acid oxidation of a ketone of the formula VI and permanganate or periodate oxidation of a diketone of the formula VII. Each of these reactions is carried out by methods well-known in the art.

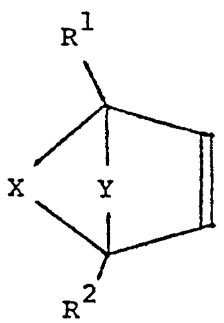

V

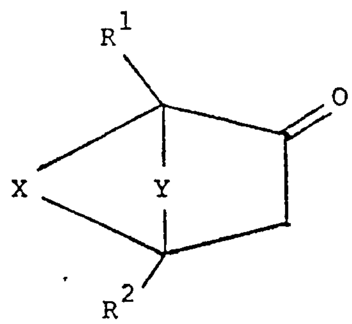

VI

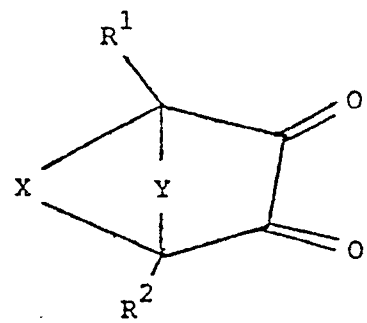

VII

For examples of preparations of specific dicarboxylic acids of the formula IV (or lower-alkyl esters thereof, which can be converted into the acids by conventional hydrolysis methods), consult: Journal of Organic Chemistry, 31, 3438 (1969); Helvetica Chimica Acta, 53, 2156 (1970); Journal of the American Chemical Society, 98, 1810 (1976); Journal of Organic Chemistry, 44, 1923 (1979); Australian Journal of Chemistry, 34, 665 (1981); and Canadian Journal of Chemistry, 59, 2848 (1981).

The amines of the formula III are prepared by known methods. Consult: United States Patent No. 4,423,049.

The compounds of the formula I are basic, and they will form acid-addition salts. All such salts are within the scope of this invention, although for administration to a human subject it is necessary to use a pharmaceutically-acceptable salt. The compounds of formula I contain more than one basic center; consequently, acid-addition salts can incorporate one or more molecules of a salt-forming acid. When more than one molecule of salt-forming acid is incorporated, the anionic counter ions can be the same or different. Acid-addition salts of a compound of the formula I are prepared by conventional methods. In a typical procedure, a compound of formula I is combined with a stoichiometric amount of an appropriate acid in an inert solvent, which can be aqueous, partially aqueous or non-aqueous. The salt is then recovered by solvent evaporation, by filtration if the salt precipitates spontaneously, or by precipitation using a non-solvent followed by filtration. Typical salts which can be prepared include sulfate, hydrochloride, hydrobromide, nitrate, phosphate, citrate, tartrate, pamoate, sulfosalicylate, methanesulfonate, benzenesulfonate and 4-toluenesulfonate salts.

As indicated hereinbefore, the compounds of formula I, wherein $R^1$, $R^2$, $R^3$, X and Y are as defined previously, and the pharmaceutically-acceptable acid-addition salts thereof, are active as antianxiety (anxiolytic) agents. This activity can be demonstrated and measured using the well-known Vogel anti-conflict test. See further, Vogel et al., Psychophamacologia, 21, 1 (1971). In a typical variation of the Vogel anti-conflict test, groups of rats are deprived of water for 48 hours, and then they are presented with an opportunity to drink water from an electrified spout. The number of times that the rats drink water (and therefore also receive an electric shock) during a 10 minute period is measured for rats which have been dosed with a test compound (treated rats). This number is compared with the number obtained for control rats, i.e., rats which have not received the test compound. An increase in the number of times that treated rats drink water, over the number of times that control rats drink water, is indicative of antianxiety activity in the compound being tested.

The antianxiety activity of the compounds of the formula I, and the pharmaceutically-acceptable acid-addition salts thereof, makes them useful for administration to humans for alleviating the symptoms of anxiety.

The compounds of the formula I, and the pharmaceutically-acceptable acid-addition salts thereof, have antidepressant properties. Antidepressant activity can be measured in rats using well-known procedures. See further, Porsolt et al., European Journal of Phamacology, 47, 379 (1978).

The antidepressant activity of the compounds of the formula I and the pharmaceutically-acceptable acid-addition salts thereof, makes them useful for administration to humans for alleviating the symptoms of depression.

A compound of formula I, or a pharmaceutically-acceptable salt thereof, can be administered to a human subject either alone, or, preferably, in combination with pharmaceutically-acceptable carriers or diluents, in a pharmaceutical composition, according to standard pharmaceutical practice. A compound can be administered orally or parenterally, which includes intravenous and intramuscular administration. However, the preferred route of administration is oral. Additionally, in a pharmaceutical composition comprising a compound of formula I, or a pharmaceutically-acceptable salt thereof, the weight ratio of carrier to active ingredient will normally be in the range from 20:1 to 1:1, and preferably 10:1 to 1:1. However, in any given case, the ratio chosen will depend on such factors as the solubility of the active component, the dosage contemplated, and the precise dosage regimen.

For oral use of a compound of this invention, the compound can be administered, for example, in the form of tablets or capsules, or as an aqueous solution or suspension. In the case of tablets for oral use, carriers which can be used include lactose and corn starch, and lubricating agents, such as magnesium stearate, can be added. For oral administration in capsule form, useful diluents are lactose and dried corn starch. When aqueous suspensions are required for oral use, the active ingredient can be combined with emulsifying and suspending agents. If desired, certain sweentening and/or flavoring agents can be added. For intramuscular and intravenous use, sterile solutions of the active ingredient can be prepared, and the pH of the solutions should be suitably adjusted and buffered. For intravenous use, the total concentration of solutes should be controlled to render the preparation isotonic.

When a compound of the present invention is to be used in a human subject, the daily dosage will be determined by the prescribing physician. In general, the dosage will depend on the age, weight and response of the individual patient, as well as the severity of the patient's symptoms. However, in most instances, an effective anxiety-alleviating amount and an effective depression-alleviating amount of a compound of the formula I, or a pharmaceutically-acceptable acid-addition salt thereof, will be from 1 to 300 mg per day, and preferably 5 to 100 mg per day, in single or divided doses. Naturally, the more active compounds of the invention will be used at the lower doses, while the less active compounds will be used at the higher doses. Also, for a given compound, an effective depression-alleviating amount will usually be greater than an effective anxiety-alleviating amount.

The following examples and preparations are being provided solely for further illustration. For nuclear magnetic resonance spectra (NMR spectra), absorptions are given in parts per million (ppm) downfield from tetramethylsilane.

EXAMPLE 1

Dextrorotatory Isomer of 3-(4-[4-(2-Pyrimidinyl)-1-piperazinyl]butyl)-1,8,8-trimethyl-3-azabicyclo[3.2.1]octan-2,4-dione.

A solution of 1.79g (7.6 mmol) of 1-(4-aminobutyl)-4-(2-pyrimidinyl)piperazine and 1.37g (7.5 mmol] of d-camphoric anhydride in 50 ml of xylene was heated under reflux for 22 hours with continuous removal of water (Dean-Stark trap). The resulting solution was cooled and evaporated, and the residue was dissolved in 25 ml of warm isopropanol. The solution was allowed to cool, and the solid which appeared was recovered by filtration. This afforded 740 mg (25% yield) of the title compound, mp 94-95 °C, $[alpha]_D^{20} = +7.1°$ (c = 10; $C_2H_5OH$).

The $^1$H-NMR spectrum of the product (250 MHz; $CDCl_3$) showed absorptions at 8.24 (d,2H,J = 4Hz), 6.41 (t,1H,J = 4Hz), 3.76 (t,4H,J = 4Hz), 3.63 (m,2H), 2.63 (d,1H), 2.40 (t,4H,J = 4Hz), 2.31 (m,2H), 2.20-2.04 (m,2H), 1.94-1.74 (m,2H), 1.74-1.6 (m,2H), 1.54-1.3 (m, 4H), 1.1 (s,3H) and 0.87 (m,6H) ppm.

The $^{13}$C-NMR spectrum (250 MHz; $CDCl_3$) showed absorptions at 178.2, 176.2, 161.7, 157.6, 109.7, 58.3, 56.6, 54.4, 53.0, 44.1, 43.7, 39.1, 34.2, 25.7, 25.0, 24.3, 22.0, 19.3 and 14.1 ppm.

The high resolution mass spectrum showed a molecular ion at $M^+$ = 399.2616. $C_{22}H_{33}N_5O_2$: requires $M^+$ = 399.2637.

Analysis: Calcd. for $C_{22}H_{33}N_5O_2$: C,66.12; H,8.34; N,17.53%. Found: C,65.91; H,8.47; N,17.47%.

EXAMPLE 2

Levorotatory Isomer of 3-(4-[4-(2-Pyrimidinyl)-1-piperazinyl]butyl)-1,8,8-trimethyl-3-azabicyclo[3.2.1]octan-2,4-dione.

Following substantially the procedure of Example 1, 1.27g (5.39 mmol) of 1-(4-aminobutyl)-4-(2-pyrimidinyl) piperazine was reacted with 0.98g (5.37 mmol) of 1-camphoric anhydride. The product was chromatographed on silica gel, eluting with dichloromethane/methanol (95:5), to give 0.88g (42% yield) of the title compound. After recrystallization from isopropanol/hexane, the product had mp 92-94° C, $[alpha]_D^{20}$ = -7.4° (c = 10, $C_2H_5OH$).

The $^1$H-NMR and $^{13}$C-NMR spectra of the product were essentially identical to those of the product of Example 1.

EXAMPLE 3

Racemic 3-(4-[4-(2-Pyrimidinyl)-1-piperazinyl]butyl)-1,8,8-trimethyl-3-azabicyclo[3.2.1]octan-2,4-dione.

The title compound was prepared from 2.47g (10.4 mmol) of 1-(4-aminobutyl)-4-(2-pyrimidinyl)-piperazine and 1.89g (10.3 mmol) of dl-camphoric anhydride, substantially according to the procedure of Example 1. This afforded 0.7g of product, mp 94-97° C (17% yield).

The $^1$H-NMR and $^{13}$C-NMR spectra of the product were essentially identical to those of the product of Example 1.

The high resolution mass spectrum showed a molecular ion at $M^+$ = 399.2669. $C_{22}H_{33}N_5O_2$ requires $M^+$ = 399.2637.

EXAMPLE 4

3-(4-[4-(2-Pyrimidinyl)-1-piperazinyl]butyl)-3-azabicyclo[3.2.1]octan-2,4-dione.

The title compound was prepared from 1.92g (8.2 mmol) 1-(4-aminobutyl)-4-(2-pyrimidinyl)piperazine and 1.14g (8.14 mmol) of 3-oxabicyclo[3.2.1]octan-2,4-dione, substantially following the procedure of Example 1. Yield: 1.0g (34% yield).

The $^1$H-NMR spectrum of the product (300 MHz; $CDCl_3$) showed absorptions at 8.02 (d,2H,J = 4Hz), 6.20 (t,1H,J = 4Hz), 3.56 (t,4H,J = 4Hz), 3.40 (m,2H), 2.88 (m,2H), 2.22 (t,4H,J = 4Hz), 2.12 (m,2H), 1.96-1.74 (m,3H), 1.72-1.56 (m,2H), 1.48-1.3 (m,1H) and 1.3-1.1 (m,4H) ppm.

The $^{13}$C-NMR spectrum of the product (300 MHz; $CDCl_3$) showed absorptions at 176.2, 161.4, 157.5, 109.6, 58.0, 52.8, 44.6, 43.4, 38.3, 32.3, 27.0, 25.7 and 23.8 ppm.

The high resolution mass spectrum showed a molecular ion at $M^+$ = 357.2181. $C_{19}H_{27}N_5O_2$ requires $M^+$ = 357.2167.

EXAMPLE 5

3-(4-[4-(2-Pyrimidinyl)-1-piperazinyl]butyl)-3-azabicyclo[3.3.1]nonan-2,4-dione

Following substantially the procedure of Example 1, 2.78g (11.8 mmol) of 1-(4-aminobutyl)-4-(2-pyrimidinyl)-piperazine was reacted with 1.88g (12.2 mmol) of 3-oxabicyclo[3.3.1]nonan-2,4-dione. The product was chromatographed on silica gel, eluting with dichloromethane/methanol (90:10), to give 2.04g (47% yield) of the title compound.

The $^1$H-NMR spectrum of the product (250 MHz; $CDCl_3$) showed absorptions at 8.25 (d,2H,J = 4Hz), 6.42 (t,1H,J = 4Hz), 3.88-3.68 (m,6H), 2.86-2.76 (m,2H), 2.45 (t,4H,J = 4Hz), 2.37 (m,2H), 2.2-2.07 (m,1H), 2.05-1.88 (m,2H) and 1.74-1.1 (9H,m) ppm.

The $^{13}$C-NMR spectrum of the product (250 MHz; $CDCl_3$) showed absorption at 175.5, 161.7, 157.7, 109.8, 58.3, 53.0, 43.5, 39.3, 38.5, 28.3, 26.2, 24.2 and 19.5 ppm.

The high resolution mass spectrum showed a molecular ion at $M^+$ = 371.2293. $C_{20}H_{29}N_5O_2$ requires $M^+$ = 371.2324.

EXAMPLE 6

3-(4-[4-(2-Pyrimidinyl)1-piperazinyl]butyl)-8,8-dimethyl-3-azabicyclo[3.2.1]octan-2,4-dione

Following substantially the procedure of Example 1, 0.54g (2.34 mmol) of 1-(4-aminobutyl)-4-(2-pyrimidinyl)piperazine was reacted With 0.36g (2.14 mmol) of 8,8-dimethyl 3 oxabicyclo[3.2.1]octan-2,4-dione. The product was chromatographed on silica gel, eluting with dichloromethane/methanol (96:4), to

give 0.25g (32% yield) of the title compound.

The $^{1}H$-NMR spectrum of the product (300 MHz; $CDCl_3$) showed absorptions at 8.12 (d,2H,J = 3Hz), 6.30 (t,1H,J = 3Hz), 3.66 (t,4H,J = 4Hz), 3.51 (m,2H), 2.50 (m,2H), 2.32 (t,4H,J = 4Hz), 2.23 (m, 2H), 2.16-2.02 (m,2H), 1.74-1.6 (m,2H), 1.37 (m,4H), 0.96 (s,3H) and 0.84 (s,3H) ppm.

The $^{13}C$-NMR spectrum of the product (300 MHz; $CDCl_3$) showed absorptions at 176.4, 157.6, 109.7, 58.2, 54.8, 53.0, 43.5, 42.0, 38.5, 26.4, 25.6, 24.1, 23.8 and 20.9 ppm.

The high resolution mass spectrum showed a molecular ion at $M^+$ = 385.2427. $C_{21}H_{31}N_5O_2$ requires 385.2480.

EXAMPLE 7

3-(4-[4-(2-Pyrimidinyl)-1-piperazinyl]butyl)-1,5-dimethyl-3-azabicyclo[3.2.1]octan-2,4-dione

Following substantially the procedure of Example 1, 4.2g (17.8 mmol) of 1-(4-aminobutyl)-4-(2-pyrimidinyl)-piperazine was reacted with 2.99g (17.8 mmol) of 1,5-dimethyl-3-oxabicyclo[3.2.1]octan-2,4-dione. The product was chromatographed on silica gel, eluting with dichloromethane/methanol (96:4), to give 1.4g (21% yield) of the title compound.

The $^{1}H$-NMR spectrum of the product (300 MHz; $CDCl_3$) showed absorptions at 8.01 (d,2H,J = 4Hz), 6.20 (t,1H,J = 4Hz), 3.57 (m,4H), 3.45 (m,2H), 2.22 (m,4H), 2.13 (m,2H), 1.70-1.54 (m,5H), 1.38-1.2 (m,5H) and 1.10 (s,6H) ppm.

The $^{13}C$-NMR spectrum of the product (300 MHz; $CDCl_3$) showed absorptions at 178.1, 161.4, 157.5, 109.6, 58.1, 52.8, 49.0, 46.5, 43.4, 39.1, 35.8, 25.7, 23.9 and 20.3 ppm.

The high resolution mass spectrum showed a molecular ion at $M^+$ = 385.2484. $C_{21}H_{31}N_5O_2$ requires $M^+$ = 385.2480.

EXAMPLE 8

3-(4-[4-(2-Pyrimidinyl)-1-piperazinyl]butyl)-3-azabicyclo[3.2.2]nonan-2,4-dione

An intimate mixture of 0.5g (2.14 mmol) of 1(4-aminobutyl)-4-(2-pyrimidinyl)piperazine and 0.22g (1.42 mmol) of 3-oxabicyclo[3.2.2]nonan-2,4-dione was heated in an oil bath at 220-230°C for 15 minutes. An additional 0.22g (1.42 mmol) of 3-oxabicyclo[3.2.2]nonan-2,4-dione was added. Heating in an oil bath at 220-230°C was continued for 15 minutes and then an additional 0.22g (1.42 mmol) of 3-oxabicyclo[3.2.2]-nonan-2,4-dione was added. Heating was continued for 30 minutes at 220-230°C and then the reaction mixture was cooled. The resulting product was chromatographed on silica gel, eluting with dichloromethane/methanol (96:4 followed by 94:6), to give 46 mg of the title compound (6% yield).

The $^{1}H$-NMR spectrum of the product (300 MHz; $CDCl_3$) showed absorptions at 8.26 (d,2H,J = 4Hz) 6.46 (t,1H,J = 4Hz) , 3.90 (t,4H,J = 3Hz), 3.72 (t,2H,J = 4Hz), 3.04 (m,2H), 2.68 (t,4H,J = 3Hz), 2.56 (t,2H,J = 4Hz), 1.95-1.7 (m,8H) and 1.64-1.42 (m,4H) ppm.

The $^{13}C$-NMR spectrum of the product (300 MHz; $CDCl_3$) showed absorptions at 178.9, 161.4, 157.7, 110.2, 57.6, 52.2, 43.1, 42.4, 41.5, 25.8, 22.7 and 21.8 ppm.

The high resolution mass spectrum showed a molecular ion at $M^+$ = 371.2316. $C_{20}H_{29}N_5O_2$ requires $M^+$ = 371.2323.

EXAMPLE 9

3-(4-[4-(2-Pyrimidinyl)-1-piperazinyl]butyl-8-methyl-3-azabicyclo[3.2.1]octan-2,4-dione can be prepared from 1-(4-aminobutyl)-4-(2-pyrimidinyl)piperazine and 8-methyl-3-oxabicyclo[3.2.1]octan-2,4-dione, substantially following the procedure of Example 1.

3-(4-[4-(2-Pyrimidinyl)-1-piperazinyl]butyl)-8,8-tetramethylene-3-azabicyclo[3.2.1]octan-2,4-dione can be prepared from 1-(4-aminobutyl)-4-(2-pyrimidinyl)piperazine and 8,8-tetramethylene-3-oxabicyclo[3.2.1]octan-2,4-dione, substantially following the procedure of Example 1.

3-(4-[4-(4-Fluoro-2-pyrimidinyl)-1-piperazinyl]butyl)-1, 8,8-trimethyl-3-azabicyclo[3.2.1.]octan-2,4-dione can be prepared from 1-(4-aminobutyl)-4-(4-fluoro-2-pyrimidinyl)-piperazine and 1,8,8-trimethyl-3-oxabicyclo-[3.2.1]octan-2,4-dione, substantially according to the procedure of Example 1.

3-(4-[4-(2-Pyrimidinyl)-1-piperazinyl]butyl)-3-azabicyclo[3.2.1]oct-6-en-2,4-dione can be prepared from 1-(4-aminobutyl)-4-(2-pyrimidinyl)piperazine and 3-oxabicyclo[3.2.1]oct-6-en-2,4-dione, substantially following the procedure of Example 1.

3-(4-[4-(2-Pyrimidinyl)-1-piperazinyl]butyl-6-methyl-3-azabicyclo[3.2.1.]octan-2,4-dione can be prepared from 1-(4-aminobutyl)-4-(2-pyrimidinyl)piperazine and 6-methyl-3-oxabicyclo[3.2.1]octan-2,4-dione, substantially following the procedure of Example 1.

3-(4-[4-(2-Pyrimidinyl)-1-piperazinyl]butyl)-6,6-dimethyl-3-azabicyclo[3.2.1]octan-2,4-dione can be prepared from 1-(4-aminobutyl)-4-(2-pyrimidinyl)piperazine and 6,6-dimethyl-3-oxabicyclo[3.2.1]octan-2,4-dione, substantially following the procedure of Example 1.

3-(4-[4-(4-Fluoro-2-pyrimidinyl)-1-piperazinyl]butyl)-3-azabicyclo[3.2.1]oct-6-en-2,4-dione can be prepared from 1-(4-aminobutyl)-4-(4-fluoro-2-pyrimidinyl)piperazine and 3-oxabicyclo[3.2.1]oct-6-en-2,4-dione, substantially following the procedure of Example 1.

EXAMPLE 10

Mono-hydrochloride Salt of the Dextrorotatory Isomer of 3-(4-[4-(2-Pyrimidinyl)-1-piperazinyl]butyl)-1,8,8-trimethyl-3-azabicyclo[3.2.1]octan-2,4-dione

To a refluxing solution of 1.04g (2.6 mmol) of the dextrorotatory isomer of 3-(4-[4-(2-pyrimidinyl)-1-piper-azinyl]butyl)-1,8,8-trimethyl-3-azabicyclo[3.2.1]octan-2,4-dione in 5 ml of isopropanol was added 0.55 ml (2.65 mmol) of 4.8N aqueous hydrochloric acid. The resulting solution was allowed to cool to room temperature, and then the volume was reduced to 3 ml by evaporation in vacuo. An additional 5 ml of isopropanol was added and the solution was heated to the boiling point. The solution was allowed to cool slowly, with stirring. The solid which appeared was collected by filtration, to give 0.64g (56% yield) of the title salt, mp 207-08° C.

The $^{13}C$-NMR spectrum (250 MHz; $D_2O$) showed absorptions at 182.4, 180.8, 161.3, 159.6, 113.0, 57.3, 55.7, 52.3, 45.4, 42.2, 39.4, 34.8, 25.8, 25.1, 22.04, 22.0, 19.3 and 14.3 ppm.

Analysis: Calcd. for $C_{22}H_{33}N_5O_2$.HCl: Cl, 8.13%. Found: Cl, 8.02%.

PREPARATION 1

3-Oxabicyclo[3.2.2]nonan-2,4-dione

A solution of 2.9g of cis-cyclohexane-1,4-dicarboxylic acid in 25 ml of acetic anhydride was heated under reflux for 2.5 hours. The solvent was removed by evaporation in vacuo, and the residue was triturated under diethyl ether. several times. The residue was dried under high vacuum to give 2.5g (97% yield) of the title compound.

The $^{13}C$-NMR spectrum of the product (300 MHz; $CDCl_3$) showed absorptions at 170.5, 41.4 and 25.2 ppm.

The $^{1}H$-NMR spectrum of the product (300 MHz; $CDCl_3$) showed absorptions at 2.24-1.34 (m,8H) and 2.5 (m,2H) ppm.

PREPARATION 2

3-Oxabicyclo[3.2.1]octan-2,4-dione

A solution of 1.63g of cis-cyclopentane-1,3-dicarboxylic acid in 8.2 ml of acetic anhydride was heated in an oil bath at ca. 100° C for 45 minutes. The excess acetic anhydride and acetic acid were removed by evaporation in vacuo to give a solid which was triturated under diethyl ether. This afforded 1.14g (79% yield) of the title anhydride.

The $^{13}C$-CMR spectrum of the product (300 MHz; $CDCl_3$) showed absorptions at 170.1, 41.8, 31.1 and 26.4 ppm.

The $^{1}H$-NMR spectrum of the product (300 MHz; $CDCl_3$) showed absorptions at 3.2 (m,2H), 2.36-1.9 (m,8H) and 1.82-1.66 (m,2H) ppm.

PREPARATION 3

1,8,8-Trimethyl-3-oxabicyclo[3.2.1]octan-2,4-dione (1-Camphoric Anhydride)

The product of Preparation 5 was cyclized with acetic anhydride, using the procedure of Preparation 2, to give 1.0g (97% yield) of the title compound.

PREPARATION 4

**cis-Cyclohexane-1,4-dicarboxylic Acid**

A steady stream of ozone was passed through a mixture of 15g (0.14 mole) of bicyclo[2.2.2]oct-2-ene and 300 ml of methanol at -70° C for 4 hours. The ozone stream was stopped, the mixture was allowed to

warm to room temperature, and then the solvent was removed by evaporation *in vacuo*. The residue was dissolved in 72 ml of formic acid, and to the resulting solution was added, cautiously, portionwise, 50 ml of 30% hydrogen peroxide. The resulting mixture was heated to 70°C, at which point an exothermic reaction took place. After the exothermic reaction had subsided, the reaction mixture was heated under reflux for 1 hour. Concentration of the reaction mixture *in vacuo* afforded a colorless oil, which was partitioned between 300 ml of ethyl acetate and 300 ml of water. The pH was adjusted to 9.5 using 2N sodium hydroxide. The aqueous phase was removed, acidified to pH 2.0, and extracted with fresh ethyl acetate. The ethyl acetate extract was dried ($Na_2SO_4$) and evaporated *in vacuo*. The residue was recrystallized from water to give 3.0g (13% yield) of cis-cyclohexane-1,4-dicarboxylic acid.

The $^{13}C$-NMR spectrum of the product (300 MHz; $CDCl_3$) showed absorptions at 177.1, 39.8 and 25.2 ppm.

PREPARATION 5

**1,2,2-Trimethyl-*cis*-cyclopentane-1,3-dicarboxylic Acid (1-Camphoric Acid)**

A solution of 1.0g (7.2 mmol) of 1,7,7-trimethylbicyclo[2.2.1]hept-2-ene in 20 ml of methanol was cooled to - 75°C, and a steady stream of ozone was passed through the solution for 1 hour. Excess ozone was removed using a stream of nitrogen, and then the reaction mixture was warmed to room temperature and evaporated *in vacuo*. The residue was dissolved in 10 ml of formic acid, and 5 ml of 30% hydrogen peroxide was added portionwise.
The reaction mixture was heated to *ca.* 75°C, at which point an exothermic reaction took place. After the exothermic reaction had subsided, the resulting mixture was heated under reflux for 1.5 hours. The cooled mixture was evaporated *in vacuo* to give 1.1g (78% yield) of the title compound.

PREPARATION 6

1,7,7-Trimethylbicyclo[2.2.1]hept-2-ene

1,7,7-Trimethylbicyclo[2.2.1]heptan-2-one (1-camphor) was converted into its 4-toluenesulfonylhydrazone, which was then reacted with methyllithium in ether, to give the title compound. The method used was that described by Shapiro and Duncan, *Organic Synthesis*, Vol 51, pp 67-69, for the conversion of racemic camphor into racemic 1,7,7-trimethylbicyclo[2.2.1]hept-2-ene.

**Claims**

**Claims for the following Contracting States : AT, BE, CH, DE, FR, GB, IT, LUX, NL, SE**

1. A bridged bicyclic imide compound of the formula

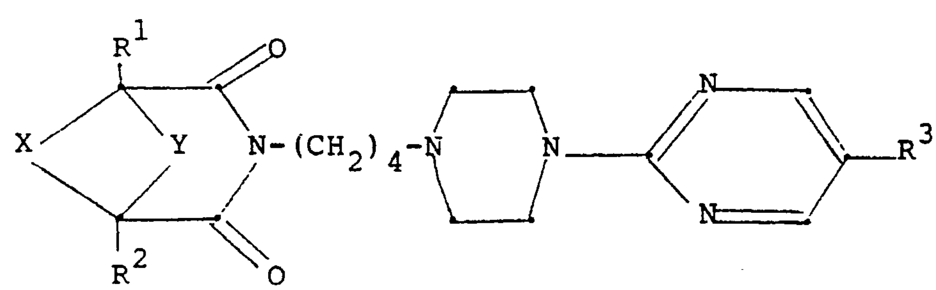

and the pharmaceutically-acceptable acid-addition salts thereof, wherein
$R^1$ and $R^2$ are each H or $CH_3$;
$R^3$ is H or F; and either
(a) X is $CH_2$, $CH_2CH_2$ or $CH_2CH_2CH_2$; and Y is $CH_2$, $CH(CH_3)$, $C(CH_3)_2$, $C(CH_2)_4$ or $CH_2CH_2$; or
(b) X is CH=CH, $CH_2CH(CH_3)$ or $CH_2C(CH_3)_2$; and Y is $CH_2$.

2. A compound according to claim 1, wherein $R^3$ is H; X is $CH_2$, $CH_2CH_2$ or $CH_2CH_2CH_2$; and Y is $CH_2$, $CH(CH_3)$, $C(CH_3)_2$, $C(CH_2)_4$ or $CH_2CH_2$.

3. A compound according to claim 2, wherein X is $CH_2CH_2$.

**4.** A compound according to claim 3, wherein $R^1$ and $R^2$ are each H and Y is $CH_2$.

**5.** A compound according to claim 3, wherein $R^1$ is $CH_3$, $R^2$ is H and Y is $C(CH_3)_2$.

**6.** The dextrorotatory isomer of 3-(4-[4-(2-pyrimidinyl)-1-piperazinyl]butyl)-1,8,8-trimethyl-3-azabicyclo-[3.2.1]octan-2,4-dione, a compound according to claim 5.

**7.** A compound according to claim 1, wherein $R^1$ is $CH_3$, $R^2$ is H, $R^3$ is F, X is $CH_2CH_2$ and Y is $C(CH_3)_2$.

**8.** A compound according to claim 1, wherein $R^3$ is H; X is $CH=CH$, $CH_2CH(CH_3)$ or $CH_2C(CH_3)_2$; and Y is $CH_2$.

**9.** A compound according to claim 8, wherein $R^1$ and $R^2$ are each H and X is $CH_2CH(CH_3)$.

**10.** A pharmaceutical composition which comprises a pharmaceutically-acceptable carrier and a bridged bicyclic imide compound according to claim 1.

**Claims for the following Contracting States : ES, GR**

**1.** A process for the preparation of a bridged bicyclic imide compound of the formula

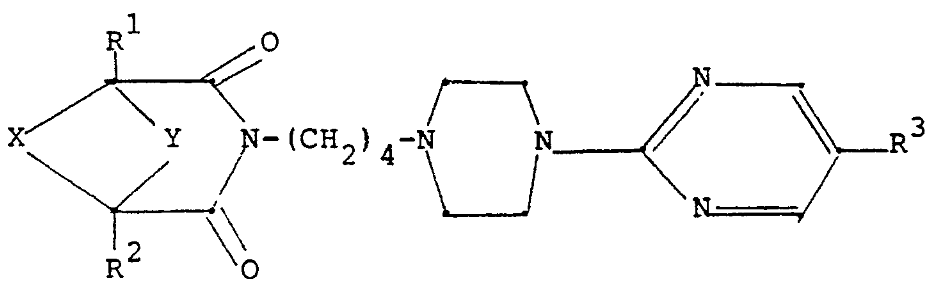

or a pharmaceutically-acceptable acid-addition salt thereof, wherein
$R^1$ and $R^2$ are each H or $CH_3$;
$R^3$ is H or F; and either
(a) X is $CH_2$, $CH_2CH_2$ or $CH_2CH_2CH_2$; and Y is $CH_2$, $CH(CH_3)$, $C(CH_3)_2$, $C(CH_2)_4$ $CH_2CH_2$; or
(b) X is $CH=CH$, $CH_2CH(CH_3)$ or $CH_2C(CH_3)_2$; and Y is $CH_2$.
characterized in that a cyclic anhydride of the formula

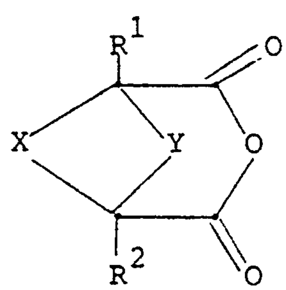

is reacted with an amine of the formula

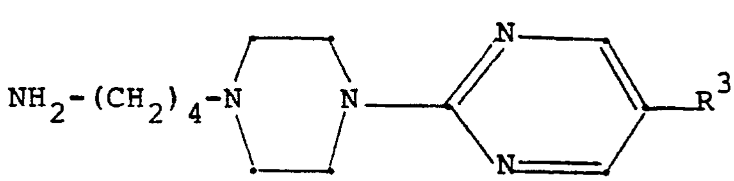

2. The process according to claim 1, wherein the process is carried out by heating substantially equimolar quantities of the anhydride and the amine at a temperature from 90 to 160°.

3. The process according to claim 2, wherein the anhydride and the amine are heated in a reaction-inert solvent.

4. The process according to claim 3, wherein the reaction-inert solvent is a hydrocarbon, such as benzene, toluene, xylene or decalin, or a methyl or ethyl ether of ethylene glycol, propylene glycol or diethylene glycol.

5. The process according to any of claims 1 to 4, wherein $R^1$ is $CH_3$, $R^2$ is H, $R^3$ is H, X is $CH_2CH_2$ and Y is $C(CH_3)_2$.

6. The process according to any of claims 1 to 4 for the preparation of the dextrorotatory isomer of 3-(4-[4-(2-pyrimidinyl)-1-piperazinyl]butyl)-1,8,8-trimethyl-3-azabicyclo[3.2.1]octan-2,4-dione.

**Revendications**
**Revendications pour les Etats contractants suivants : AT, BE, CH, DE, FR, GB, IT, LI, LU, NL, SE**

1. Imide bicyclique ponté, de formule

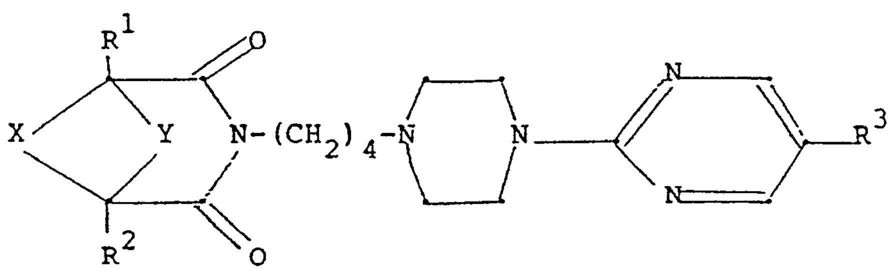

et ses sels d'addition d'acides pharmaceutiquement acceptables, formule dans laquelle
$R^1$ et $R^2$ représentent chacun H ou un groupe $CH_3$ ;
$R^3$ représente H ou F ; et
(a) X représente un groupe $CH_2$, $CH_2CH_2$ ou $CH_2CH_2CH_2$ ; et Y représente un groupe $CH_2$, $CH(CH_3)$, $C(CH_3)_2$, $C(CH_2)_4$ ou $CH_2CH_2$ ; ou bien
(b) X représente un groupe $CH{=}CH$, $CH_2CH(CH_3)$ ou $CH_2C(CH_3)_2$ ; et Y représente un groupe $CH_2$.

2. Composé suivant la revendication 1, dans lequel $R^3$ représente H ; X représente un groupe $CH_2$, $CH_2CH_2$ ou $CH_2CH_2CH_2$ ; et Y représente un groupe $CH_2$, $CH(CH_3)$, $C(CH_3)_2$, $C(CH_2)_4$ ou $CH_2CH_2$.

3. Composé suivant la revendication 2, dans lequel X représente un groupe $CH_2CH_2$.

4. Composé suivant la revendication 3, dans lequel $R^1$ et $R^2$ représentent chacun H et Y représente un groupe $CH_2$.

5. Composé suivant la revendication 3, dans lequel $R^1$ représente un groupe $CH_3$, $R^2$ représente H et Y représente un groupe $C(CH_3)_2$.

6. Isomère dextrogyre de la 3-(4-[4-(2-pyrimidinyl)-1-pipérazinyl]butyl)-1,8,8-triméthyl-3-azabicyclo[3.2.1]-octane-2,4-dione, représentant un composé suivant la revendication 5.

7. Composé suivant la revendication 1, dans lequel $R^1$ représente un groupe $CH_3$, $R^2$ représente H, $R^3$ représente F, X représente un groupe $CH_2CH_2$ et Y représente un groupe $C(CH_3)_2$.

8. Composé suivant la revendication 1, dans lequel $R^3$ représente H ; X représente un groupe $CH{=}CH$, $CH_2CH(CH_3)$ ou $CH_2C(CH_3)_2$ ; et Y représente un groupe $CH_2$.

9. Composé suivant la revendication 8, dans lequel $R^1$ et $R^2$ représentent chacun H et X représente un groupe $CH_2CH(CH_3)$.

10. Composition pharmaceutique, qui comprend un support pharmaceutiquement acceptable et un imide bicyclique ponté suivant la revendication 1.

**Revendications pour les Etats contractants suivants : ES, GR**

1. Procédé de préparation d'un imide bicyclique ponté, de formule

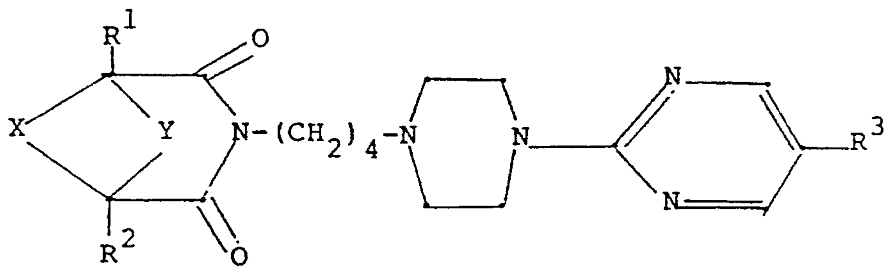

ou d'un de ses sels d'addition d'acides pharmaceutiquement acceptables, formule dans laquelle
$R^1$ et $R^2$ représentent chacun H ou un groupe $CH_3$ ;
$R^3$ représente H ou F ; et
(a) X représente un groupe $CH_2$, $CH_2CH_2$ ou $CH_2CH_2CH_2$ ; et Y représente un groupe $CH_2$, $CH(CH_3)$, $C(CH_3)_2$, $C(CH_2)_4$ ou $CH_2CH_2$ ; ou bien
(b) X représente un groupe $CH=CH$, $CH_2CH(CH_3)$ ou $CH_2C(CH_3)_2$ ; et Y représente un groupe $CH_2$ ;
caractérisé en ce qu'un anhydride cyclique de formule

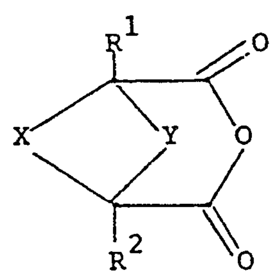

est amené à réagir avec une amine de formule

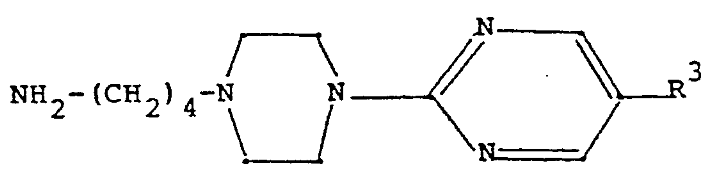

2. Procédé suivant la revendication 1, qui est mis en oeuvre par chauffage de quantités pratiquement équimolaires de l'anhydride et de l'amine à une température de 90 à 160 °C.

3. Procédé suivant la revendication 2, dans lequel l'anhydride et l'amine sont chauffés dans un solvant inerte vis-à-vis de la réaction.

4. Procédé suivant la revendication 3, dans lequel le solvant inerte vis-à-vis de la réaction est un hydrocarbure, tel que le benzène, le toluène, le xylène ou la décaline, ou bien un éther méthylique ou

éthylique d'éthylèneglycol, de propylèneglycol ou de diéthylèneglycol.

5. Procédé suivant l'une quelconque des revendications 1 à 4, dans lequel $R^1$ représente un groupe $CH_3$, $R^2$ représente H, $R^3$ représente H, X représente un groupe $CH_2CH_2$ et Y représente un groupe $C(CH_3)_2$.

6. Procédé suivant l'une quelconque des revendications 1 à 4, pour la préparation de l'isomère dextrogyre de la 3-(4-[4-(2-pyrimidinyl)-1-pipérazinyl]-butyl)-1,8,8-triméthyl-3-azabicyclo[3.2.1]octane-2,4-dione.

**Patentansprüche**
**Patentansprüche für folgende Vertragsstaaten : AT, BE, CH, DE, FR, GB, IT, LI, LU, NL, SE**

1. Verbrückte bicyclische Imidverbindung der Formel

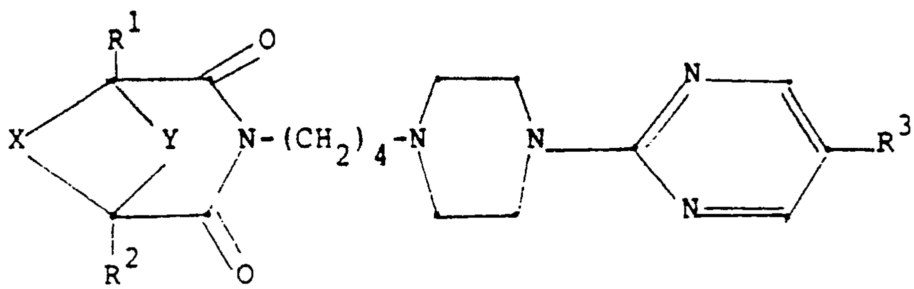

und die pharmazeutisch annehmbaren Säure-Additionssalze davon, worin
$R^1$ und $R^2$ jeweils H oder $CH_3$ sind,
$R^3$ H oder F ist
und entweder
(a) X $CH_2$, $CH_2CH_2$ oder $CH_2CH_2CH_2$ ist und Y $CH_2$, $CH(CH_3)$, $C(CH_3)_2$ $C(CH_2)_4$ oder $CH_2CH_2$ ist, oder
(b) X CH = CH, $CH_2CH(CH_3)$ oder $CH_2C(CH_3)_2$ ist und Y $CH_2$ ist.

2. Verbindung gemäß Anspruch 1, worin $R^3$ H ist, X $CH_2$, $CH_2CH_2$ oder $CH_2CH_2CH_2$ ist und Y $CH_2$, $CH(CH_3)$, $C(CH_3)_2$, $C(CH_2)_4$ oder $CH_2CH_2$ ist.

3. Verbindung gemäß Anspruch 2, worin X $CH_2CH_2$ ist.

4. Verbindung gemäß Anspruch 3, worin $R^1$ und $R^2$ jeweils H sind und Y $CH_2$ ist.

5. Verbindung gemäß Anspruch 3, worin $R^1$ $CH_3$ ist, $R^2$ H ist und Y $C(CH_3)_2$ ist.

6. Das rechtsdrehende Isomere von 3-(4-[4-(2-Pyrimidinyl)-1-piperazinyl]butyl)-1,8,8-trimethyl-3-azabicyclo[3.2.1]octan-2,4-dion, eine Verbindung gemäß Anspruch 5.

7. Verbindung gemäß Anspruch 1, worin $R^1$ $CH_3$ ist, $R^2$ H ist, $R^3$ F ist, X $CH_2CH_2$ ist und Y $C(CH_3)_2$ ist.

8. Verbindung gemäß Anspruch 1, worin $R^3$ H ist, X CH = CH, $CH_2CH(CH_3)$ oder $CH_2C(CH_3)_2$ ist und Y $CH_2$ ist.

9. Verbindung gemäß Anspruch 8, worin $R^1$ und $R^2$ jeweils H sind und X $CH_2CH(CH_3)$ ist.

10. Pharmazeutische Zusammensetzung, die einen pharmazeutisch annehmbaren Träger und eine verbrückte bicyclische Imidverbindung gemäß Anspruch 1 umfaßt.

**Patentansprüche für folgende Vertragsstaaten : ES, GR**

1. Verfahren zum Herstellen einer verbrückten bicyclischen Imidverbindung mit der Formel

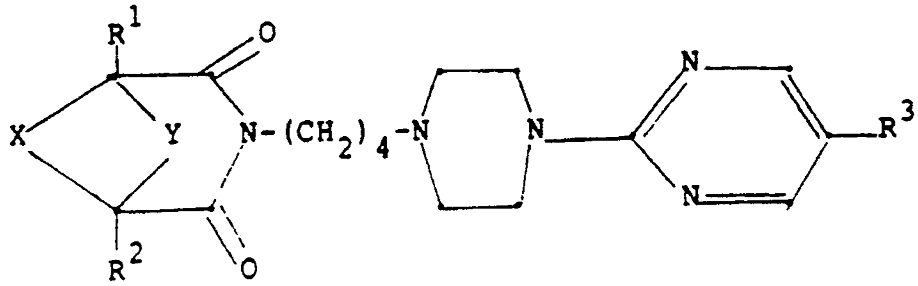

oder eines pharmazeutisch annehmbaren Säure-Additionssalzes davon, worin

$R^1$ und $R^2$ jeweils H oder $CH_3$ sind,

$R^3$ H oder F ist

und entweder

(a) X $CH_2$, $CH_2CH_2$ oder $CH_2CH_2CH_2$ ist und Y $CH_2$, $CH(CH_3)$, $C(CH_3)_2$ $C(CH_2)_4$ oder $CH_2CH_2$ ist, oder

(b) X $CH=CH$, $CH_2CH(CH_3)$ oder $CH_2C(CH_3)_2$ ist und Y $CH_2$ ist,

dadurch gekennzeichnet, daß ein cyclisches Anhydrid mit der Formel

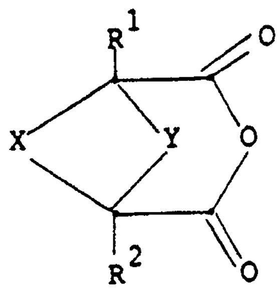

mit einem Amin mit der Formel

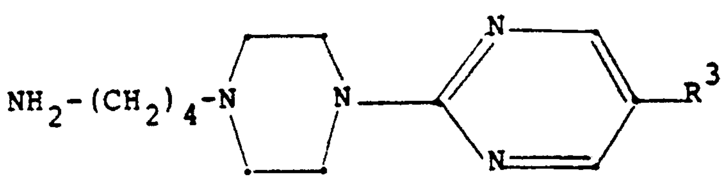

umgesetzt wird.

2. Verfahren nach Anspruch 1, worin das Verfahren ausgeführt wird, indem im wesentlichen äquimolare Mengen des Anhydrids und des Amins auf eine Temperatur von 90 bis 160° C erhitzt werden.

3. Verfahren nach Anspruch 2, worin das Anhydrid und das Amin in einem reaktionsinerten Solvens erhitzt werden.

4. Verfahren nach Anspruch 3, worin das reaktionsinerte Solvens ein Kohlenwasserstoff wie Benzol, Toluol, Xylol oder Decalin oder ein Methyl- oder Ethylether von Ethylenglycol, Propylenglycol oder Diethylenglycol ist.

5. Verfahren nach einem beliebigen der Ansprüche 1 bis 4, worin $R^1$ $CH_3$ ist, $R^2$ H ist, $R^3$ H ist, X $CH_2CH_2$ ist und Y $C(CH_3)_2$ ist.

6. Verfahren nach einem beliebigen der Ansprüche 1 bis 4 zum Herstellen des rechtsdrehenden Isomeren von 3-(4-[4-(2-Pyrimidinyl)-1-piperazinyl]butyl)-1,8,8-trimethyl-3-azabicyclo [3.2.1]octan-2,4-dion.